# EUROPEAN PATENT SPECIFICATION

(11) **EP 0 485 143 B1**
(45) Date of publication and mention of the grant of the patent: **17.12.1997**
(21) Application number: 91310180.4
(22) Date of filing: 04.11.1991
(51) Int. Cl.: A61K 9/127, A61K 38/46

(54) **Liposomal compositions and processes for their production**
Liposomale Mittel sowie Verfahren zu deren Herstellung
Compositions liposomales et leurs procédés de préparation

(30) Priority: 06.11.1990 PT 95812; 28.11.1990 PT 96037
(43) Date of publication of application: 13.05.1992
(73) Proprietor: INSTITUTO NACIONAL DE ENGENHARIA E TECNOLOGIA INDUSTRIAL, 1699 Lisboa Codex (PT)
(72) Inventor: Meirinhos da Cruz, Maria Eugenia, P-1500 Lisboa (PT); Santana Jorge, Joao Carlos, P-2880 Almada (PT); Figueria Martins, Maria Barbara dos Anjos, P1400 Lisboa (PT); Guilherme Gaspar, Maria Manuela de Jesus, P-1900 Lisboa (PT); Esteves Simoes, Aida da Conceicao, P-2780 Oeiras (PT); Perez-Soler, Roman, Houston, Texas 77005 (US)
(74) Representative: Powell, Stephen David

(56) References cited:
- US-A- 4 933 121
- PATENT ABSTRACTS OF JAPAN, vol. 6, no. 165 (C-121)[1043], 28th August 1982; & JP-A-57 82 311 (TANABE SEIYAKU K.K.) 22-05-1982
- CHEM. PHARM. BULL., vol. 32, no. 6, June 1984, pages 2442-2445; T. OHSAWA et al.: "A novel method for preparing liposome with a high capacity to encapsulate proteinous drugs: freeze-drying method"
- JOURNAL OF MICROENCAPSULATION, vol. 7, no. 4, October/December 1990, pages 497- 503; S.F. ALINO et al.: "High encapsulation efficiencies in sized liposomes produced by extrusion of dehydration-rehydration vesicles"

## Description

This invention relates to liposomal compositions containing enzymes having asparaginase activity.

L-Asparaginase (L-asparaginase; L-asparagine amido hidrolase E.C.3.5.1.1.) is an effective anti tumor agent active against human acute lymphoblastic leukemia (1). These malignant cells have a metabolic defect in L-asparagine synthetase being dependent on an exogenous supply of L-asparagine. The mode of action of L-asparaginase is based on starvation to death of malignant cells by degradation of their specific substrate. The intravenous administration of free enzyme causes several side effects in humans, mainly acute allergic reactions ranging from fever, skin rashes to death secondary to anaphylactic shock, thrombosis or hemorrhage (2,3).

To partially overcome these limitations, several strategies can be followed such as the inclusion of asparaginase in liposomes, with the aim of masking the immunogenicity of the enzyme and increasing its biological half-life in the organism.

Analysis of the current literature indicates that although the encapsulation of L-asparaginase in liposomes has attracted attention in the past (15, 16) and in recent years (17, 18), biologically effective systems have not been achieved either from the galenic or the pharmacological point of view.

It has been found that improved liposomal compositions are those which have the parameters and are prepared by the methods described hereinafter.

The present invention comprises a liposomal composition containing an enzyme having L-Asparaginase activity characterised by having a protein/lipid ratio of at least 30µg/µmol, the size of liposomes being up to 1000 nm, and containing a hydrophobic derivative of L-Asparaginase in the lipid phase. Preferably the size of the liposomes is no greater than 800 nm, a most advantageous size range being from 100 to 200 nm. It will be understood that compositions according to the invention consist at least predominantly of liposomes having the specified maximum size. The required liposome size may be achieved by means of filters of the appropriate pore size as is well understood in the art.

The hydrophobic derivative of the native enzyme which is located in the lipid phase may be an acylated derivative having an acyl group containing from 8 to 18 carbon atoms such as palmitoyl.

Preferably the compositions of the present invention also containing native L-asparaginase in the aqueous phase.

In order to ensure the desired proportions of protein and lipid in the final formulations it is desirable, when preparing the liposomes, to start with an initial protein/lipid ratio of at least 40 µg/µmol (0.06 mg protein/1 mg lipid) or 80 µg/µmol (0.11 mg protein/ 1 mg lipid), respectively for hydrophobised or native enzyme.

Liposome compositions may be prepared, according to this invention, by a general process which will be explained hereinafter in flow sheet diagrammatic form. The process is of interest for the formulation of hydrophobised proteins including hydrophobised asparaginase. The general process is adaptable depending on whether native enzyme as well as hydrophobised enzyme is used. In general the process comprises forming multilamellar liposomes containing the enzyme and subjecting the liposomes to lyophilisation, rehydration and extrusion under pressure. For hydrophobised enzyme the multilamellar liposomes are formed as empty liposomes and a hydrophobic L-Asparaginase derivative is added as dry solid to the liposomes before the lyophilisation stage. For preferred compositions the process is characterised by forming multilamellar liposomes from an aqueous solution of the enzyme and the lipid, lyophilising the resulting liposomes, rehydrating the lyophilised liposomes by treatment with a small volume of non-saline suspending fluid followed, after a resting phase, by treatment with a saline solution, diluting the resulting suspension and extruding the suspension under pressure without prior separation of non-incorporated enzyme.

Liposome compositions and processes for their preparation by this invention are characterised by the use of any of the following lipids, hydrogenated or not, individually or in mixtures, in a molar ratio from 0.01 to 10: distearoylphosphatidylcholine (DSPC), phosphatidylcholine (PC), cholesterol (Chol) or its derivatives, sphyngomyelin (SM), dioleoylphosphatidylcholine (DOPC), dioleoylphosphatidylglycerol (DOPG), phosphatidylglycerol (PG), dimirystoylphosphatidylcholine (DMPC), dipalmitoylphosphatidylcholine (DPPC), gangliosides ceramides, phosphatidylinositol (PI), phosphatidic acid (PA), dicetylphosphate (DcP), dimirystoylphosphatidylglycerol (DMPG), stearylamine (SA), dipalmitoylphosphatidylglycerol (DPPG) and other synthetic lipids.

The improved liposomal formulations of L-asparaginase activity makes use of extruded vesicles (VET) . Although showing smaller encapsulation parameters than multilamellar vesicles (DRV), the liposomes prepared by the present process are highly effective to degrade circulating substrates as, similarly to other kind of small vesicles (19), VET liposomes have been found to circulate for more prolonged periods of time than the multilamellar vesicles. No data are available in the prior literature for the encapsulation of native L-asparaginase in VET.

According to the present invention liposomes are prepared by the dehydration and rehydration of multilamellar vesicles (MLV) containing intra and extra liposomal L-Asparaginase in a solution of 0.3 OSM. The so formed liposomes are then resuspended in a volume 20 times higher than the final volume and submitted to several extrusions through polycarbonate filters of pore size from 5.0 to 0.1 µm or lesser. To wash the extraliposomal material and to concentrate liposomes, an ultracentrifugation is carried out in solutions of desired osmolarity, generally 0.3 OSM, obtaining the final liposomal preparation in the desired volume.

The so prepared extruded vesicles show a high retention of specific enzymatic activity (73 to 99%) and Encapsulation Efficiency (E.E.) up to 28%, according to lipid composition and size. The P/L ratio in liposomes is at least 32 µg/ µmol of lipid, being also size and composition dependent (Table I).

The higher stability in human serum observed for VET containing L-Asparaginase when compared with DRV elucidates the importance of size. In fact, the stability in human plasma of liposomes type DRV was increased from 65 to 95% after size reduction by extrusion. Although it seems important to avoid the existence of osmotic gradients after liposome in vivo administration, little attention has been given up to now, to the effects of solute osmolarity on protein encapsulation. The method usually described in literature of using 0.154 M NaCl to keep osmolarity, resulted in a decrease in the E.E. with all lipid compositions tested. By rehydration of liposomes with mannitol, as done by the present method, there was no effect on all the parameters under study. The liposomes are easily centrifuged and physiological osmolarity can be kept after reconstitution.

Liposomal formulations of L-Asparaginase herein described are significantly less toxic than free L-Asparaginase as concluded by the increase of animals survivals from 83 to 100%, when both forms of enzyme are administered (Table II). The present formulations of L-Asparaginase show enhanced antitumoral activity as compared to free enzyme or to multilamellar liposomes (DRV), also tested (Table IV). These results are according to the pharmacokinetic data shown in Table III: liposomes prepared by the present invention increases 4.5 to 14.5 fold the half life of free enzyme, depending on the lipid composition used. The fact that the encapsulated enzyme circulates longer than the free one increases the pharmacological effects continually degrading the circulating substrate L-asparagine.

According to the present invention, the incorporation of the modified protein is carried out by the addition of dried hydrophobized L-asparaginase to multilamellar liposomes (MLV) obtained by drying under nitrogen stream the necessary amount of lipid followed by hydration of the lipidic film to form MLV. Dehydration and rehydration in solutions with osmolarity of 0.3 OSM is the next step. The so formed liposomes are then resuspended in a volume 20 times higher than the final volume and submitted to several extrusions through polycarbonate filters of pore size from 5.0 to 0.1 um or lesser. An ultracentrifugation in gradients, with a composition being dependent of the lipidic composition of the liposomes, allows to collect liposomes in the middle of the gradient and neglecting the non-incorporated protein that accumulates at the bottom of the gradient. To wash the gradient substance out of the liposomal media, a last ultracentrifugation is carried out in solutions of desired osmolarity, generally 0.3 OSM, obtaining the final liposomal preparation in the desired volume.

In prior methods described in the literature, the addition of modified protein is done by means of a solution to a film of dried lipids forming liposomes with encapsulated protein (20). Some authors do a lyophilization of these solutions followed by a hydration (21). These processes are substantially different from that of the present invention because, in the present process, the protein addition is carried out in a lyophilized form into preformed liposomes. Also, no other author uses the ultracentrifugation technique in gradients to separate the non-incorporated protein. There are no data in the literature concerning extrusion techniques applied to hydrophobic proteins incorporated in liposomes. The results of the present process are much improved in terms of recovery, encapsulation efficiency, protein concentration and biological activity. By this process, liposomes with great catalytic activity and great circulating time are obtained. Furthermore, Acyl-L-Asparaginase has not hitherto been encapsulated in such large amount of final protein per mass of lipid organized in liposomes.

Small unilamellar liposomes prepared by sonication of multilamellar vesicles have been explored in the past as carriers of hydrophobic derivatives of L-asparaginase by superficial insertion of the acylated enzyme in the outer layer of liposomes, but without success (22). The efficiency of the process was extremely low (incorporation efficiency from 3 to 4%), leading to preparations unsuitable for intravenous administration, as high doses were needed to reach an acceptable antitumor activity.

Incorporation of hydrophobized L-Asparaginase has also been tried by us using several other prior methods namely:
1 - Incubation of small unilamellar vesicles in the presence of different concentrations of protein and different amounts and kinds of detergent. We obtained a maximal incorporation efficiency of 3% and recovery of less than 1 % of the protein initially present in the process.
2 - Use of the detergent-dialysis method for preparing large unilamellar vesicles, a method extensively used by different authors to incorporate membrane proteins in the lipid matrix of liposomes (23). With this process, and trying different amounts and kinds of detergent, and different initial protein concentrations and lipids, we obtained a maximal incorporation efficiency of 10% concomitantly with an extremely low recovery, around (0.5 to 3%), of the initial protein used in the process.
3 - Use of large unilamellar vesicles obtained by the reverse evaporation method (24), comparatively using several different protein concentrations and different conditions to remove the solvent. With this method we obtained a maximal incorporation efficiency of less than 1%. with a recovery of the initial protein used in the process smaller than 0.5%.
4 - Use of multilamellar vesicles obtained by the dehydration rehydration method as described in the literature (15). We obtained a maximal incorporation efficiency of 40% (21).

The liposomes, according to the present invention, besides their high incorporation efficiency from 50 to 98% have the capability of having 50 to 75% of the total catalytic activity in the intact form of the vesicles.

Pharmacokinetic studies clearly indicate a longer circulating time for our liposomal preparations irrespectively of the lipidic composition used, when compared to Palmitoyl-L-ASNase. Immunological response studies have provided evidence for the ability of PC:CHOL:PI liposomes to avoid the response of the immune system to the modified enzyme as shown for other systems with native asparaginase or other enzymes (15). Antitumoral studies strongly evidence the ability of the liposomes obtained by the present process to be active against tumor models in animals.

The present invention also comprises DRV and VET having in their structure simultaneously both forms of the enzyme: L-asparaginase and hydrophobized L-asparaginase. Vesicles of this kind are prepared by adding multilamellar vesicles containing native L-asparaginase to dried hydrophobized L-asparaginase. All the following steps necessary to obtain the final preparation are similar to the ones described for incorporation of hydrophobized L-asparaginase. The resulting formulations are novel and show improved and sustained release of the enzyme in vivo.

The methods, vesicle size and lipid composition here used, resulted in high E.E., high in vitro stability and no in vitro toxicity. Higher intraliposomal concentration and stability in plasma result in higher circulation time of the enzyme and greater efficiency, as the target is a circulating substrate. The encapsulation method is extremely simple and easy to scalle up. Namely: using enzyme free MLV or MLV containing intra and extra liposomal enzyme; mannitol in the rehydration step; high volume of dilution of DRV before filtration; non washed DRV as starting material to prepare VET; abolishing the freeze-thaw steps refered in previous methods (9); using small size vesicles and lipidic mixtures of PC:Chol:SA; PC:Chol:PI and PC:Chol: GM1.

The liposomal forms of L-Asparaginase of the present invention are immunogenic safe by intravenous injection and evidence higher therapeutic activity.

### METHOD

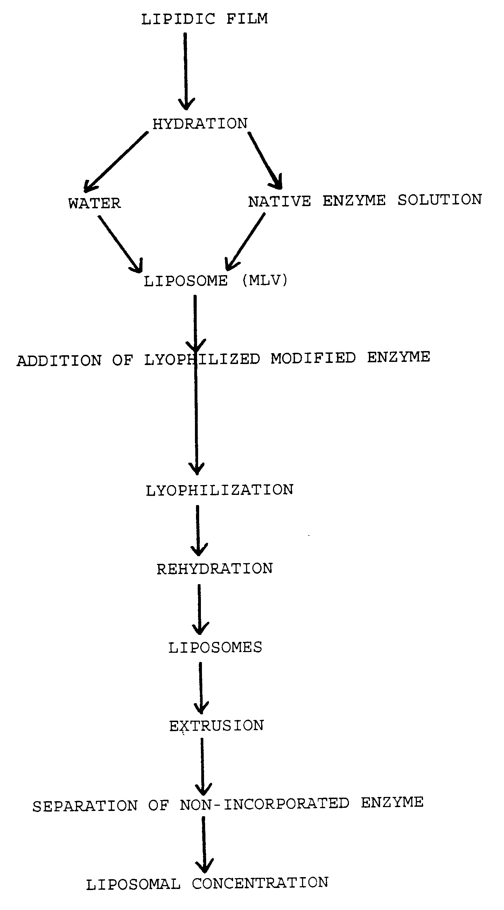

The liposomal product is prepared according to a general scheme for the incorporation either of the hydrophobic form of L-Asparaginase, alone or together with the native form of L-Asparaginase. A general scheme of the method is presented as Figure 3 followed by detailed description in text.

The method consists on the following steps:
1. The lipid mixture is dried.
2. The lipidic film is hydrated with water or an aqueous solution containing protein.
3. The formed multilamellar liposomes, alone or mixed with lyophilized protein, are dehydrated.
4. The resulting powder is rehydrated with an aqueous solution.
5. The suspension is filtered under pressure.
6. The non-encapsulated material is separated from liposomes by ultracentrifugation, in a gradient medium if necessary.
7. The final liposomes are concentrated by ultracentrifugation.

According to protein solubility, options a) or b) are used:
a) In case of the hydrophobized protein:
   - the lipidic film is hydrated with water.
   - the addition of protein to lipid is done, in a lyophilized form, in step 3.
   - the non-incorporated material is ultracentrifugated in a gradient medium if necessary.
b) In case of a mixture of native and hydrophobized protein:
   - The lipidic film is hydrated with an aqueous solution containing protein.
   - The formed liposomes (MLV), mixed with lyophilized protein, are dehydrated.
   - The non-encapsulated material is separated from liposomes by ultracentrifugation, in a gradient medium if necessary.

Method for incorporation of hydrophobized form of L-Asparaginase:

The method consists of the following steps:
1. The lipid mixture (1 - 100 mg/ml) is dried under vacuum in a N2 stream;
2. The lipidic film is hydrated with 1ml of water, slowly, with gentle stirring to form multilamellar vesicles.
3. The multilamellar vesicles are added to protein in lyophilized form (0,2 - 100 mg); the protein to lipid ratio should be higher than 40 µg/ (0.06 mg protein / 1 mg lipid). This suspension is gently mixed until the powder of protein is homogeneously dispersed into the suspension of liposomes.
4. The mixture is lyophilized on a freeze-drier under vacuum (25 mtorr during, at least, 4 hours).
5. Rehydration of the lyophilized powder is performed in two steps: firstly with 100 ul to 250 ul of a solution of osis with vigorous vortexing followed by 30 min rest at a temperature 10 C above the phase transition temperature of the lipid, secondly the volume is brought up to the initial 1 ml volume with 0.154 M NaCl.
6. The resulting mixture is then 10 fold diluted with 0.154M NaCl and extruded under pressure through polycarbonate filters of controlled pore size, successively 5.0 µm, 2.0 µm, 1.0 µm, 0.8 µm, 0.6 µm, 0.4 µm and 0.2 µm, stopping on the filter pore size necessary to produce liposomes of the required diameter, using an Extruder device.
7. The extruded mixture is ultracentifuged at 45,000 rpm during 45 min in a sacarose gradient, collecting the liposomes at the middle of the gradient and neglecting the protein in the bottom of the gradient. To wash the sacarose out of the liposomes and to concentrate the liposome preparation a final ultracentrifugation, at 270.000 g during 60 min, is done. The pellet, consisting of liposomes containing incorporated hydrophobized L-Asparaginase is resuspended in 1 ml of 0.154 M NaCl

Method for incorporation of both native and hydrophobized form of L-Asparaginase:

The methods for incorporation of both forms of L-Asparaginase is identical to the method of incorporation of the hydrophobized L-Asparaginase with a single difference: in step 2, instead of hydrating the lipidic film with water, the hydration is carried out with an aqueous solution of L-Asparaginase (0.2 - 2 mg/ml).

### CHEMICAL AND BIOLOGICAL CHARACTERIZATION

Here are presented chemical and biological characterizations of this invention, for extruded liposomes (type VET) made from different lipid compositions, as compared with those liposome obtained by another technique (DRV Dehydration-Rehydration Vesicles).

The yield of the process means the percentage of liposomal to initial protein concentration. Protein was estimated by Lowry method (25), after previous disruption of liposomes with Triton X and SDS.

Encapsulation efficiency (E.E.) means the ratio of the liposomal and the initial protein/lipid, in percentage. The lipidic concentration was estimated according to Fiske and Subbarow (26). The enzymatic activity was estimated according to Jayaram et al (27).

### B. HYDROPHOBIZED FORM OF L-ASPARAGINASE

### 1. - Encapsulation of hydrophobized L-Asparaginase in liposomes

The first step to incorporate palmitoyl-L-ASNase was to determine the liposomal composition and the size of such vesicles. We found that the best formulations were PC:Chol:PI in a molar ratio of 10:5:1 and PC:Chol:SA in a molar ratio of 7:2:0.25. Incorporation parameters were determined for these type of vesicles submitted to the extrusion procedure (VET 200) and non-submitted to the extrusion step (DRV). The recovery ranged from 36+-8 to 43+-15% to the VET 200 and from 56+-9 to 65+-7 % to the DRV. The encapsulation efficiency ranged from 69+-19 to 74+-11% (VET 200) and from 80+-16 to 86+-14 % (DRV). The ratio of intact activity to total (destroyed) activity is the most significant value as it indicates the activity of palmitoyl-L-ASNase exposed to the outer medium. It increases from about 30% in the DRV to 51 and 74 % in the VET 200 formulations.

In order to obtain enough amount of liposomal palmitoyl-L-ASNase to the animal studies it became necessary to increase the quantity of lipid and protein in the liposomal preparations. For that, the initial ratio of 16.5 umol/0.25 mg (lipid/protein) was modified to 66.0 umol/2.87 mg. For the VET 200 liposomes made with PC:Chol:PI, the new incorporation parameters were 29 +-7 % for the recovery, 46+-6 % for the encapsulation efficiency, and 54+-14 % for the Intact Activity/Total activity. For the VET 200 liposomes made with PC:Chol:SA, the new incorporation parameters were 44+-6 % for the recovery, 66+-10 % for the encapsulation efficiency, and 46+-12 % for the Intact Activity/Total activity.

### 2. -Pharmacokinetics

Pharmacokinetics studies were done in Charles River mice.

Blood was collected from rectroorbital vein at fixed times.

Activity of L-Asparaginase was determined in total blood.

The pharmacokinetics parameters of the VET 200 and of palmytoil-L-ASNase suspended in Tween 80 0.5% filtered through 0.2 um were obtained from Figure 2 and are shown in Table I. The incorporation of palmitoyl-L-ASnase in liposomes increased the mean residance time for 4.1 H to values greater than 32 H.

The Area Under Curve has also inceased significantly, from 77.4 U.H/ml to values greater than 650 U.H/ml. The plasma clearence has been reduced with the incorporation of liposomes, from 0.646 ml/H to values smaller than 0.076 ml/H. No alteration has been observed in the volume of distribution (steady-state) with the incorporation.

These pharmacokinetic studies clearly indicate a longer circulating time for the liposomal preparations independently of the lipidic composition used, when compared to Palmitoyl-L-ASNase.

### 3. - In vivo toxicity

Toxicity studies of formulations obtained by the present process, revealed not to generate immunological response in sensitized mice. Groups of Charles River mice were immunized with three IM injections of free or liposomal enzymes on day 0, 10 and 20. On day 30, animals were challenged with an IV injection of 2000 U/Kg of free or liposomal enzymes.

The immunological studies (results presented in Table II) show that palmitoyl-L-ASNase retains part of the toxicity of the native L-ASNase, in either the suspensions used, with 4 of the 6 animals showing anaphylatic shock and in one animal causing dead.

The liposomal formulation of PC:Chol:PI improved these results. When it is used as the sensitizating and the challenge agent, no anaphylatic shock or dead was observed. Also in the case of palmitoyl-L-ASNase as the challenge agent, animals sensitized with PC:Chol:PI liposomes show no anaphylatic shock or dead.

By another way, liposomal formulation of PC:Chol:SA have increased immunological response in either the cases, where these liposomes are used as both the sensitizing and challenge agent (all the 6 animals showing evidence of anaphylatic shock with 2 of then going to dead), and where these liposomes are used only as the sensitizing agent and Palmitoyl-L-ASNase in Tween 80 0.5% used as the challenging agent ( all the 6 animals showing evidence of anaphylatic shock and 3 of them going to dead).

The immunological response studies evidenced for the ability of PC:CHOL:PI liposomes to avoid the response of the immune system to the modified enzyme as shown for other systems with native asparaginase or other enzymes (15). PC:CHOL:SA liposomes increase the immune response when compared to the non-liposomal enzyme, despite of the antitumoral activity that these type of vesicles maintain.

### 4. - Antitumour Activity

As a crucial test to evaluate the therapeutic importance of the product, in vivo studies were performed using BDF1 mice.

Table III compares the antitumor activity of Palmitoyl-L-ASNase in Tween 80 and of the liposomal formulations, estimated as their ability to supress the growth of solid lymphoma P1534. The activity of all preparations is dose dependent. 400 U/kg is a non effective dose. The liposomal Palmitoyl-L-ASNase formulations is as effective as the free Palmitoyl-L-ASNase in the 2000 U/kg dose, both exhibiting antitumor activity, with a T/C > 700%. All the dead animals suffered of liver, pancreas and spleen metastasis with no native tumor. Histopathological studies of the liver, spleen and pancreas of the dead animals revealed the massive presence of tumor cells with almost total absence of normal cells in these organs. This is an indication that, even in the low doses, all formulations were able to suppress growth of native tumor.

**TABLE I**

| PHARMACOKINETIC STUDIES Pharmacokinetic parameters obtained by mathematical treatment of Figure 2. Mean residence time (MRT), area under curve (AUC total), plasmatic clearence (Clp), volume of distribution at the steady state (Vol. D.(SS)) and half life (T 1/2) are shown for each experiment. Dose equal to 2000 U/Kg of weight. | | | | | |
|---|---|---|---|---|---|
| | MRT (H) | AUC (U.H/ml) | Clp (ml/H) | Vol.D.(SS) (ml) | T 1/2 (H) |
| Palmitoyl-L-ASNase in Tween 80 0.5% | 4.1 | 77.4 | 0.646 | 2.64 | 2.88 |
| Pc:Chol:SA VET 200 | 32.1 | 656.3 | 0.076 | 2.44 | 24.3 |
| Pc:Chol:PI VET 200 | 33.1 | 740.9 | 0.067 | 2.24 | 23.7 |

**TABLE II**

| THE EFFECT OF LIPOSOMAL COMPOSITION ON IMMUNOLOGICAL RESPONSE The effect of liposomal formulation in the immunological response. Anaphylatic shock was noticed by means of behavior alterations | | | |
|---|---|---|---|
| Experiment | Animals | Shock | Dead |
| IM-Free L-Asparaginase | 6 | 6 (100%) | 1 (17%) |
| IV-Free L-Asparaginase | | | |
| IM-Acyl-L-ASNase, Tween 80 0.5% | 6 | 4 (66%) | 1 (17%) |
| IV-Acyl-L-ASNase, Tween 80 0.5% | | | |
| IM-Acyl-L-ASNase, 0.154 M NaCl | 6 | 4 (66%) | 1 (17%) |
| IV-Acyl-L-ASNase, 0.154 M NaCl | | | |
| IM-Acyl-L-ASNase, PC:Chol:PI(0.2 um) | 6 | 0 (0%) | 0 (0%) |
| IV-Acyl-L-ASNase, PC:Col:PI(0.2 um) | | | |
| IM-Acyl-L-ASNase, Pc:Col:PI(0.2 um) | 6 | 0 (0%) | 0 (0%) |
| IV-Acyl-L-ASNase, Tween 80 0.5% | | | |
| IM-Acyl-L-ASNase, Tween 80 0.5% | 6 | 0 (0%) | 0 (0%) |
| IV-Acyl-L-ASNase, PC:Col:PI(0.2 um) | | | |
| IM-Acyl-L-ASNase, Tween 80 0.5% | 6 | 0 (0%) | 0 (0%) |
| IV-Acyl-L-ASNase, PC:Chol:SA(0.2 um) | | | |
| IM-Acyl-L-ASNase, PC:Chol:SA(0.2 um) | 6 | 6 (100%) | 2 (33%) |
| IV-Acyl-L-ASNase, PC:Chol:SA(0.2 um) | | | |
| IM-Acyl-L-ASNase, PC:Chol:SA(0.2 um) | 6 | 6 (100%) | 3 (50%) |
| IV-Acyl-L-ASNase, Tween 80 0.5% | | | |

**TABLE III**

| Antitumor activity of liposomal formulations incorporating Palmitoyl-L-Asparaginase and of Palmitoyl-L-Asparaginase solubilized in Tween 80 0.5% | | |
|---|---|---|
| Experiment | Animals cured | %T/C |
| | | |

| Palmitoyl-L-ASNase | | |
|---|---|---|
| 2000 U/Kg | 9 of 10 | >700 |
| 400 U/Kg | 5 of 10 | >700 |

| Liposomal Palmitoyl-L-ASNase | | |
|---|---|---|
| PC:CHOL:PI 0.8 um | | |
| 2000 U/Kg | 7 of 9 | >700 |
| 400 U/Kg | 4 of 8 | >700 |

| PC:CHOL:SA 0.2 um | | |
|---|---|---|
| 2000 U/Kg | 7 of 9 | >700 |
| 400 U/Kg | 4 of 8 | >700 |

| PC:CHOL:PI 0.2 um | | |
|---|---|---|
| 2000 U/Kg | 7 of 9 | >700 |
| 400 U/Kg | 4 of 8 | >700 |

### BIBLIOGRAPHY:

(1) Wriston J. C. JR., "L-Asparaginase", in: "The enzymes", Cap. 5 pp. 101-121, ed. by: Boyer P. D., Academic Press, New York, 1976.
(2) Clarkson B., Krakoff I., Burchenal J., Karnofsky D., Golbey R., Dowling M., Oettgen H., Lipton A., (1970), "Clinical results of treatment with E. Coli L-ASNase in adults with leukemia, lymphoma, and solid tumors", Cancer, 25, 2: 279-305.
(3) Capizzi R. L., Powell B. L., Cooper M. R., Stuart J. J., Muss H.B., Richards II F., Jackson D.V., White D. R., Spurr C. L. Zekan P. J., Cruz J. M., Craig J. B., (1985), "Sequential high-dose Ara-C and Asparaginase in the therapy of previously treated and untreated patients with acute leukemia", Seminars in Oncology, 12, 2, 3: 105-113.
(4) Perez-Soler R., Khokhar A. R., Hacker M. P., Lopez-Berestein G., (1986), "Toxicity and antitumor activity of cis-bis-cyclopentenecarboxylato-1,2-diaminocyclohexane Platinum (II) encapsulated in multilamellar vesicles", Cancer Res., 46:6269-6273.
(5) Lopez-Berestein G., Fainstein V., Hopfer R., Mehta K., Sullivan M. P., Keating M., Rosenblum M.G., Mehta R., Luna M., Hersh E. M., Reuben J., Juliano L., Bodey G.P., (1985) "Liposomal Amphotericin B for the treatment of systemic fungal infections in patients with cancer: a preliminary study", J. Infect. Dis., 151, 4: 704-710.
(6) Vadiei K., Lopez-Berestein G., Perez-Soler R., Luke D. R., (1989), "In vitro evaluation of liposomal cyclosporine", Int. J. Pharmac., 57: 133-138.
(7) Gabizon A., Sulkes A., Peretz T., Druckmann S., Goren D., Amselem S., Barenholz Y., (1989) "Liposome-associated doxorubicin: preclinical pharmacology and exploratory clinical phase", UCLA Symposia on Molecular and Cellular Biology - New Series in "Liposomes in the therapy of infectious diseases and cancer", Alan R. Liss, Inc., 89: 391-402.
(8)Eppstein D.A., Longenecker J.P., (1988) "Alternative delivery systems for peptides and proteins as drugs", CRC Crit. Rev. Therap. D. Sys. 5, 2 : 99-139.
(9) Kirby C., Gregoriadis G.(1984) "Dehydration-rehydration vesicles: a simple method for high yield drug entrapment in liposomes", Biotecnhol., Nov.: 979-984.
(10) Eppstein D.A., Marsh Y. V., Van Der Pas M., Felgner P. L., Schreiber A. B., (1985), "Biological activity of liposome-encapsulated murine interferon is mediated by a cell membrane receptor", Proc. Natl. Acad. Sci. USA, 82: 3688-3692.
(11) Eppstein D. A., (1986), "Medical utility of interferons: approaches to increasing therapeutic efficacy", Pharm.Int., pp.1985.
(12) Eppstein D. A., (1988), "Pragmatic approaches to delivery of peptides and proteins as drugs" in:"Targeting of drugs: anatomical and physiological considerations" ed. by: Gregoriadis G., Poste G., NATO ASI Series A: Life Sciences Vol. 155, pp. 189-202, Plenum Press, New York, 1988
(13) Ullman E. F., Tarnowski T., Felgner P., Gibbons I., (1987), "Use of liposome encapsulation in a combined single liquid reagent for homogeneous enzyme immunoassay", J. Clin. Chem., 33: 1579.
(14) Gregoriadis G., Davis D., Davies A., (1987), "Liposomes as immunological adjuvants: antigen incorporation studies", Vaccine, 5: 145-151.
(15) Neerunjun D., Gregoriadis G., (1976), "Tumor regression with liposome - entrapped L-ASNase: some immunological advantages", Biochem. Soc. Trans. 4: 135-136.
(16) Fishman Y., Citri N. (1975), "L-Asparaginase entrapped in liposomes: preparation and properties", Febs Lett. 60,1: 17-20.
(17) Michailin V.S., Kondrashin A.A., Berezov T. T., (1986), "Peculiarities of immobilization and catalytic properties of native commercial L-Asparaginase in liposomes formed from soybean phospholipids", Vopr. Med. Khim, 32: 68-72.
(18) Ohsawa T., Miura H., Harada Kiyoshi, (1985), "Evaluation of a new liposome preparation technique, the freeze-thawing method, using L-ASNase as a model drug", Chem. Pharm. Bull, 33: 2916-2923.
(19) Senior J. H., (1987), "Fate and behavior of liposomes in vivo: a review of controlling factors" Crit. Rev. Ther. Drug Carrier Syst., 3,2: 123-193.
(20) Koelsch R., Lasch J., Klibanov A.L., Torchilin V.P., (1981), "Incorporation of chemically modified proteins into liposomes", Acta Biol. Med. Germ., 40: 331-335.
(21) Martins M.B.F., Jorge J.C.S., Cruz M.E.M., (1990), "Acylation of L-asparaginase with total retention of enzymatic activity", Biochimie, 72: 671-675.
(22) Claassen E., Rooijen N., (1983), "A comparative study on the effectiveness of various procedures for attachement of two proteins (L-asparaginase and horse radish peroxidase) to the surface of liposomes" Prep. Biochem., 13: 167-174.
(23) Goldmacher V.S. (1983) "Immobilization of protein molecules on liposomes", Biochem. Pharm. 32: 1207-1210.
(24) Weissig V., Lasch J., Klibanov A.L., Torchilin V.P., (1986) "A new hydrophobic anchor for the attachment of proteins to liposomal membranes", FEBS Lett., 202: 86-90.
(25) Lowry O. H., Rosenbrough N. J., Farr A. L., Randall R. J., (1951) "Protein measurement with the Folin phenol reagent" J. Biol. Chem., 193:265-275.
(26) Fiske C. H., Subbarow Y. (1925) J. Biol. Chem., 66: 375-400.
(27) Jayaram H. N., Cooney D. A., Jayaram S., (1974) "A simple and rapid method for the estimation of L-Asparaginase in chromatographic and electrophoretic effluents: comparison with other methods" Anal. Biochem., 59: 327-346.

## Claims

1. A liposomal composition containing an enzyme having L-Asparaginase activity characterized by having a protein/lipid ratio of at least 30 µg/umol, the size of liposomes being up to 1000 nm, and containing a hydrophobic derivative of L-Asparaginase in the lipid phase.

2. A composition according to claim 1 in which the size of liposomes is up to 800 nm.

3. A composition according to claim 2 in which the size of the liposomes is from 100 to 200 nm.

4. A composition according to claim 1, 2 or 3 in which the derivative of L-Asparaginase is an acylated derivative having an acyl group containing from 8 to 18 carbon atoms.

5. A composition according to claim 4 in which the derivative is Palmitoyl-L-Asparaginase.

6. A composition according to any of claims 1 to 5, also containing native L-Asparaginase in the aqueous phase.

7. A process of preparing a liposomal composition according to any of claims 1 to 5, which comprises forming multilamellar liposomes, adding a hydrophobic L-Asparaginase derivative as dry solid to the liposomes and subjecting the liposomes to lyophilization, rehydration and extrusion under pressure.

8. A process according to claim 7 in which the solid derivative is added in the form of lyophilized material.

9. A process according to claim 7 or 8, in which the multilamellar liposomes are first formed with native L-Asparaginase in their aqueous phase.

10. A process according to any of claims 7 to 9, in which any enzymic material not incorporated into the liposomes is removed before or after the extrusion stage.

11. A process according to claim 10, in which any non-incorporated enzyme is separated from the reconstituted liposomes by centrifugation in a gradient if necessary.

12. A process of incorporation of a hydrophobic protein into liposomes characterized by adding the protein in dry form to preformed multilamellar liposomes which are either empty liposomes or contain a hydrophilic protein in their aqueous phase, lyophilizing the mixture and rehydrating the lyophilized material to reconstitute liposomes, and filtering under pressure to reduce size.

13. Liposome compositions when prepared by a process according to any of claims 7 to 12.

14. Liposome compositions and processes for their preparation according to any of the preceding claims characterised by the use of any of the following lipids, hydrogenated or not, individually or in mixtures, in a molar ratio from 0.01 to 10: distearoylphosphatidylcholine (DSPC), phosphatidylcholine (PC), cholesterol (Chol), or its derivatives, sphingomyelin (SM), dioleoylphosphatidylcholine (DOPC), dioleoylphosphatidylglycerol (DOPG), phosphatidylglycerol (PG), dimirystoylphosphatidylcholine (DMPC), dipalmitoylphosphatidylcholine (DPPC), gangliosides, ceramides, phosphatidylinositol (PI), phosphatidic acid (PA), dicetylphosphate (DcP), dimirystoylphosphatidylglycerol (DMPG), stearylamine (SA), dipalmitoylphosphatidylglycerol (DPPG) and other synthetic lipids.

## Patentansprüche

1. Liposomale Zusammensetzung, die ein Enzym mit L-Asparaginaseaktivität enthält, dadurch gekennzeichnet, daß sie ein Protein/Lipid-Verhältnis von wenigstens 30 µg/µmol aufweist, daß die Größe der Liposomen bis zu 1000 nm beträgt und ein hydrophobes Derivat der L-Asparaginase in der Lipidphase enthalten ist.

2. Zusammensetzung nach Anspruch 1, bei der die Größe der Liposomen bis zu 800 nm beträgt.

3. Zusammensetzung nach Anspruch 2, bei der die Größe der Liposomen 100 bis 200 nm beträgt.

4. Zusammensetzung nach Anspruch 1, 2 oder 3, bei der das Derivat der L-Asparaginase ein acyliertes Derivat ist mit einer Acylgruppe, die 8 bis 18 Kohlenstoff-Atome enthält.

5. Zusammensetzung nach Anspruch 4, bei der das Derivat Palmitoyl-L-Asparaginase ist.

6. Zusammensetzung nach irgendeinem der Ansprüche 1 bis 5, die auch native L-Asparaginase in der wässrigen Phase enthält.

7. Verfahren zur Herstellung einer liposomalen Zusammensetzung nach irgendeinem der Ansprüche 1 bis 5, welches folgendes aufweist: Bildung multilamellarer Liposomen, Beigeben eines hydrophoben L-Asparaginase-Derivates als Trockensubstanz zu den Liposomen und Behandlung der Liposomen mittels Lyophilisation, Rehydration und Extrusion unter Druck.

8. Verfahren nach Anspruch 7, bei dem das Feststoff-Derivat in Form einer lyophilisierten Substanz hinzugefügt wird.

9. Verfahren nach Anspruch 7 oder 8, bei der die multilamellaren Liposomen zuerst mit nativer L-Asparaginase in ihrer wässrigen Phase gebildet werden.

10. Verfahren nach irgendeinem der Ansprüche 7 bis 9, bei dem irgendeine den Liposomen nicht inkorporierte enzymatische Substanz vor oder nach der Extrusionsstufe entfernt wird.

11. Verfahren nach Anspruch 10, bei dem irgendein nicht inkorporiertes Enzym von den rekonstituierten Liposomen durch Gradientenzentrifugation, falls erforderlich, abgetrennt wird.

12. Verfahren zum Inkorporieren eines hydrophoben Proteins in die Liposomen dadurch gekennzeichnet, daß das Protein in Trockenform zu vorgebildeten multilamellaren Liposomen hinzugefügt wird, die entweder leere Liposomen sind oder die ein hydrophiles Protein in ihrer wässrigen Phase enthalten, daß die Mischung lyophilisiert und das lyophilisierte Material zur Rekonstitution der Liposomen rehydratisiert wird, und daß unter Druck gefiltert wird zur Reduzierung der Größe.

13. Liposomen-Zusammensetzung hergestellt nach einem Verfahren gemäß irgendeinem der Ansprüche 7 bis 12 hergestellt.

14. Liposomen-Zusammensetzung und Verfahren zu ihrer Präparation nach irgendeinem der vorausgehenden Ansprüche dadurch gekennzeichnet, daß irgendeins der folgenden Lipide, hydriert oder nicht, einzeln oder in Mischungen, in einem molaren Verhältnis von 0,01 bis 10 verwendet wird: Distearoylphosphatidylcholin (DSPC), Phosphatidylcholin (PC), Cholesterol (Chol) oder seine Derivate, Sphingomyelin (SM), Dioleoylphosphatidylcholin (DOPC), Dioleoylphosphatidylgycerol (DOPG), Phosphatidylgycerol (PG), Dimyristoylphosphatidylcholin (DMPC), Dipalmitoylphosphatidylcholin (DPPC), Ganglioside, Ceramide, Phosphatidylinositol (PI), Phosphatidsäure (PA), Dicetylphosphat (DcP), Dimyristoylphosphatidylgycerol (DMPG), Stearylamin (SA), Dipalmitoylphosphatidylglycerol (DPPG) und andere synthetische Lipide.

## Revendications

1. Composition liposomale contenant un enzyme d'activité L-Asparaginase, caractérisée en ce qu'elle comprend un rapport protéine/lipide d'au moins 30 µg/µmol, une dimension de liposomes allant jusqu'à 1000 nm, et contient un dérivé hydrophobe de L-Asparaginase dans la phase lipidique.

2. Composition suivant la revendication 1, dans laquelle la dimension des liposomes va jusqu'à 800 nm.

3. Composition suivant la revendication 2, dans laquelle la dimension des liposomes est de 100 à 200 nm.

4. Composition suivant la revendication 1, 2 ou 3, dans laquelle le dérivé de L-Asparaginase est un dérivé acylé ayant un groupe acyle contenant de 8 à 18 atomes de carbone.

5. Composition suivant la revendication 4, dans laquelle ledit dérivé est la palmitoyl-L-Asparaginase.

6. Composition suivant l'une quelconque des revendications 1 à 5, caractérisée en ce qu'elle contient aussi de la L-Asparaginase native dans la phase aqueuse.

7. Procédé de préparation d'une composition liposomale selon l'une quelconque des revendications 1 à 5, ledit procédé comprenant la formation de liposomes multilamellaires, l'addition d'un dérivé hydrophobe de L-Asparaginase, en tant que solide sec, à des liposomes et soumission des liposomes à lyophilisation, réhydratation et extrusion sous pression.

8. Procédé suivant la revendication 7, dans lequel le dérivé solide est ajouté sous la forme d'un matériau lyophilisé.

9. Procédé suivant la revendication 7 ou 8, dans lequel les liposomes multilamellaires sont formés en premier avec la L-Asparaginase native dans leur phase aqueuse.

10. Procédé suivant l'une quelconque des revendication 7 à 9, dans lequel tout matériau enzymatique non-incorporé dans les liposomes est éliminé avant ou après l'étape d'extrusion.

11. Procédé suivant la revendication 10, dans lequel tout enzyme non-incorporé est séparé des liposomes reconstitués par centrifugation sous gradient si nécessaire.

12. Procédé d'incorporation d'une protéine hydrophobe dans des liposomes, caractérisé par l'addition de la protéine sous forme sèche aux liposomes multilamellaires préformés qui sont soit des liposomes vides ou contiennent une protéine hydrophile dans leur phase aqueuse, la lyophilisation du mélange et la réhydratation du matériau lyophilisé pour reconstituer les liposomes, et la filtration sous pression pour réduire leur dimension.

13. Compositions de liposomes préparées selon le procédé de l'une quelconque des revendications 7 à 12.

14. Compositions de liposomes et procédés pour leur préparation selon l'une quelconque des revendications précédentes, caractérisés par l'utilisation de l'un quelconque des lipides suivants, hydrogénés ou non, soit individuellement soit en mélanges, dans un rapport molaire de 0,01 à 10 : distéaroylphosphatidylcholine (DSPC), phosphatidylcholine (PC), cholestérol (Chol) ou ses dérivés, sphingomyéline (SM), dioléoylphosphatidylcholine (DOPC), dioléoylphosphatidylglycérol (DOPG), phosphatidylglycérol (PG), dimirystoylphosphatidylcholine (DMPC), dipalmitoylphosphatidylcholine (DPPC), gangliosides, céramides, phosphatidylinositol (PI), acide phosphatidique (PA), dicétylphosphate (DcP), dimirystoylphosphatidylglycérol (DMPG), stéarylamine (SA), dipalmitoylphosphatidylglycérol (DPPG) et autres lipides synthétiques.
